# EUROPEAN PATENT SPECIFICATION

(11) **EP 3 030 229 B1**
(45) Date of publication and mention of the grant of the patent: **13.03.2019**
(21) Application number: 14834482.3
(22) Date of filing: 07.08.2014
(51) Int. Cl.: A61K 31/00, A61K 31/201, A61K 31/426, A61K 31/427, A61K 31/575, A61P 17/10, A61P 17/00

(54) **ANTI-ACNE COMPOSITIONS COMPRISING BILE ACID-FATTY ACID CONJUGATES**
ANTI-AKNE-ZUSAMMENSETZUNGEN MIT GALLENSÄURE-/FETTSÄURE-KONJUGATEN
COMPOSITIONS ANTI-ACNÉ COMPRENANT DES CONJUGUÉS ACIDES BILIAIRES-ACIDES GRAS

(30) Priority: 08.08.2013 US 201361863454 P
(43) Date of publication of application: 15.06.2016
(73) Proprietor: Galmed Research and Development Ltd., 6578317 Tel Aviv (IL)
(72) Inventor: DAYAN, Nava, Fairlawn, New Jersey 07410 (US); BAHARAFF, Allen, 6215441 Tel Aviv (IL)
(74) Representative: Korn, Richard Mervyn
(86) International application number: PCT/IL2014/050718
(87) International publication number: WO 2015/019359

(56) References cited:
- EP-A2- 0 058 000
- WO-A1-99/52932
- WO-A1-2015/019358
- ES-A1- 2 296 463
- US-A- 3 860 712
- US-A1- 2011 256 247
- LECH MRZEK ET AL: "Investigation of new acyloxy derivatives of cholic acid and their esters as drug absorption modifiers", STEROIDS, ELSEVIER SCIENCE PUBLISHERS, NEW YORK, NY, US, vol. 76, no. 10, 21 April 2011 (2011-04-21), pages 1082-1097, XP028240843, ISSN: 0039-128X, DOI: 10.1016/J.STEROIDS.2011.04.014 [retrieved on 2011-05-05]

## Description

### FIELD OF THE INVENTION

The present invention relates to pharmaceutical compositions comprising a bile acid fatty acid conjugate for use in treatment of conditions associated with altered skin sebum levels, such as Acne Vulgaris.

### BACKGROUND OF THE INVENTION

Sebum is a mixture of relatively nonpolar lipids (such as triglycerides, oils, waxes and fats) which are mostly synthesized and secreted from the sebaceous glands. Human sebaceous glands are holocrine secreting tissues which are part of the pilosebaceous units (PSUs). Sebaceous glands are connected to hair follicles and are present in most of skin surfaces. Holocrine secretions, such as sebum, result from the lysis of secretory cells in a gland. Holocrine secretions are first produced inside the secretory cells present in a gland. These secretory cells then rupture to release (secrete) the contents of these cells into the lumen, or interior space, of a gland. The sebum is normally emptied onto the skin surface through the opening of the follicle, commonly called a pore.

Secreted sebum provides a coating for the exterior surface of the skin. Although sebum normally helps lubricate, moisturize and protect the skin, its production plays a pivotal role in the pathogenesis of skin conditions such as acne and seborrhea (an abnormally increased secretion and discharge of sebum). Abnormal sebum production is known to promote the formation of comedones and increased sebum production is one of the early events that can contribute to the onset of acne. An increase in sebum secretion occurs in many people starting at about 9 years of age and continues to increase up to 17 years of age at which point the adult level of sebum secretion is typically reached. This period of increased sebum production is when most cases of acne occur.

Acne Vulgaris is one of the most treated skin condition in the United States and other countries. Acne vulgaris is a commonly referred to simply as "acne" even though many other different and clinically distinct forms of acne are known. Acne affects many adolescents and adults.

Acne vulgaris is a disease of the pilosebaceous unit of the skin. The pathogenesis of acne is complex, incompletely understood and defined by the interplay of at least the following four factors: increased sebum production by the sebaceous glands, hyper-keratinization of the upper part of the follicle, colonization of the pilosebaceous unit by Propionibacteriumacnes (P. acnes), and release of inflammatory mediators into the follicle and the surrounding epidermis.

There are four main strategies for treating acne, each strategy directed to treatment of at least one of the above-mentioned factors. One strategy is to correct an altered pattern of follicular keratinization that occurs as a result of acne. A second strategy is to decrease sebaceous gland activity and sebum production. A third strategy is to decrease the size of the follicular bacterial population and in particular to decrease the number of follicular P. acnes bacteria. A fourth strategy is to inhibit the production and/or effects of extracellular inflammatory mediators (such as cytokines and inflammatory cells). Importantly, the majority of these treatment strategies suffer from limited efficacy or undesirable side effects and thus several of these strategies may be combined for treatment.

Several categories of compositions have been used to implement these different acne treatment strategies. Isotretinoin and vitamin A derivatives represent one such category of compositions. Isotretinoin reduces sebaceous gland size by decreasing the proliferation of basal sebocytes, decreasing sebum production by up to 90% and inhibiting sebocyte differentiation. Isotretinoin is available in dosage forms suitable for either topical or oral administration. Oral administration of isotretinoin has revolutionized the treatment of severe acne. This is because isotretinoin is the first drug able to alter follicular keratinization, alter sebum production and produce anti-inflammatory effects. Unfortunately, isotretinoin is a known teratogen and can cause developmental defects. A number of other serious side effects are also associated with oral administration isotretinoin treatment. These side effects include psychiatric disorders, such as depression and psychosis, as well as intracranial hypertension, acute pancreatitis, increased blood lipid levels, hearing impairment, hepatotoxicity and inflammatory bowel disease. Topically, retinoic acid is known to be a primary irritant. Retinoids, which are commonly used for treatment of acne, have been shown to be cytotoxic for fibroblasts and epithelial cells in the range of 0.6-3*10⁵ M (Varani et al. Journal of Investigative Dermatology (1993) 101, 839-842), and to increase epithelial cell death (Ding et al. Invest Ophthalmol Vis Sci. 2013 Jun 26;54(6):4341-50).

Fatty Acid Bile Acid or Bile Salt conjugates (FABACs), referred to also as Bile Acid Fatty Acid conjugates (BAFACs), are a family of synthetic molecules that may be used to improve conditions related to bile acids or cholesterol metabolism. FABACs are believed to lower blood cholesterol concentration, reduce liver fat levels and dissolve gallstones (Gilat et al., Hepatology 2003; 38: 436-442; and Gilat et al., Hepatology 2002; 35: 597-600).

US Patents 6,384,024, 6,395,722, 6,589,946 disclose use of certain FABACs in dissolving cholesterol gallstones in bile and treating arteriosclerosis. These and additional FABACs were disclosed in US Patents 7,501,403 and US 8,110,564 as well as in US Application Publication US 2012/0214872 for use in treating fatty liver, in reducing blood cholesterol levels and in treating hyperglycemia, diabetes, insulin resistance and obesity. More recently, US Application No. 2012/0157419 disclosed FABACs as useful for treating brain diseases characterized by amyloid plaque deposits (e.g., Alzheimer's disease). Nowhere in the art is it disclosed or suggested that FABACs may be useful for prevention or treatment of skin conditions associated with altered sebum levels such as acne.

Canadian Patent Application 2,166,427 discloses use of bile acids chenodeoxycholic acid and/or ursodeoxycholic acid for the preparation of a medicament for treatment of atopic dermatitis. WO02/083147 discloses certain bile acid derivatives as Farnesoid X receptor (FXR) ligands for prevention or treatment of FXR-mediated diseases or conditions. There remains an unmet need, however, for safe and effective agents for treating or preventing conditions associated with altered sebum levels such as acne.

### SUMMARY OF THE INVENTION

The present invention relates to compositions comprising fatty acid bile acid conjugates (FABACs) for use in a method of treating a skin condition associated with altered sebum levels including, but not limited to, acne.

The present invention is based in part on the unexpected discovery that FABACs are able to reduce gene expression levels of keratin 10 and keratin 1 in skin fibroblasts, as exemplified herein below. Without wishing to be bound by any theory or mechanism, reduced expression of keratin 1 and 10 may result in reduced keratinocyte differentiation, thus hyper keratinization and follicular clogging associated with conditions such as acne may be treated using FABACs.

The present invention is further based on the surprising discovery that administration of FABACs does not result in a significant effect on viability of epidermal cells in contrast to administration of retinoids.

According to a first aspect, the present disclosure provides a method of treating a skin condition associated with altered sebum levels, the method comprises the step of administering to a subject in need thereof an effective amount of a composition comprising as an active ingredient a fatty acid bile acid conjugate (FABAC) and a pharmaceutically acceptable diluent or carrier, wherein the FABAC has the formula I:

W-X-G (I)

wherein G represents a bile acid or a bile salt radical; W represents one or two fatty acid radicals having 6-22 carbon atoms; and X represents a bonding member comprising a heteroatom or a direct C-C or C=C bond. Each possibility represents a separate embodiment of the present invention.

According to some embodiments, the composition is a pharmaceutical composition. According to some embodiments, the pharmaceutical composition is formulated for oral administration. According to certain embodiments, the pharmaceutical composition is a topical composition formulated for topical administration. According to some embodiments, the pharmaceutical composition formulated for topical administration further comprises a cosmetically acceptable diluent, carrier or excipient. According to some embodiments, topical administration refers application to the skin of a subject afflicted with a skin condition associated with altered sebum levels.

According to other embodiments, the composition is a topical composition formulated for cosmetic use. According to some embodiments, the topical composition formulated for cosmetic use is formulated for topical administration. The term "topical administration", as used herein, refers to administration through body surfaces, preferably through skin. According to some embodiments, the topical composition formulated for cosmetic use comprises a cosmetically acceptable diluent, carrier or excipient in addition to or in place of the pharmaceutically acceptable diluent or carrier. Each possibility represents a separate embodiment of the present invention.

According to some embodiments, administering relates to topical or oral administration. Each possibility represents a separate embodiment of the present invention. According to some embodiments, administering relates to oral administration.

According to some embodiments, the disclosed composition is a pharmaceutical composition comprising at least one FABAC as an active ingredient and formulated for oral administration. According to certain embodiments, the disclosed composition is a topical pharmaceutical composition formulated for topical administration, comprising at least one FABAC of the invention as an active ingredient.

According to another aspect, there is provided a composition for treating a skin condition associated with altered sebum levels, the composition comprising as an active ingredient a fatty acid bile acid conjugate (FABAC) and a pharmaceutically acceptable diluent or carrier, wherein the FABAC has the formula I:

W-X-G (I)

wherein G represents a bile acid or a bile salt radical; W represents one or two fatty acid radicals having 6-22 carbon atoms; and X represents a bonding member comprising a heteroatom or a direct C-C or C=C bond, thereby treating the skin conditions associated with altered sebum levels in said subject. Each possibility represents a separate embodiment of the present invention.

According to some embodiments, the present disclosure provides at least one fatty
acid bile acid conjugate (FABAC) for use in preparation of a medicament for treatment of a skin condition associated with altered sebum levels, wherein the FABAC has the formula I:

W-X-G (I)

wherein G represents a bile acid or a bile salt radical; W represents one or two fatty acid radicals having 6-22 carbon atoms; and X represents a bonding member comprising a heteroatom or a direct C-C or C=C bond, thereby treating the skin conditions associated with altered sebum levels in said subject. Each possibility represents a separate embodiment of the present disclosure.

According to certain embodiments, the medicament is a topical medicament formulated for topical administration. According to some embodiments, the medicament further comprises at least one pharmaceutically acceptable or cosmetically acceptable diluent, carrier or excipient. Each possibility represents a separate embodiment of the present disclosure.

According to some embodiments, the present disclosure provides a pharmaceutical composition comprising at least one FABAC as an active ingredient, wherein the pharmaceutical composition is a topical composition formulated for topical administration and further comprises at least one pharmaceutically acceptable diluent, carrier or excipient. According to some embodiments, the composition of the disclosure comprises at least one cosmetically acceptable diluent, carrier or excipient in addition to or in place of the at least one pharmaceutically acceptable diluent, carrier or excipient. Each possibility represents a separate embodiment of the present disclosure.

According to some embodiments, the skin condition associated with altered sebum levels is selected from the group consisting of: acne, dandruff, seborrhea, seborrheic dermatitis, a sebaceous cyst, sebaceous hyperplasia and a combination thereof. Each possibility represents a separate embodiment of the present invention.

According to another embodiment, said skin condition associated with altered sebum levels is acne. In a specific embodiment, said acne is acne vulgaris.

According to some embodiments, the skin condition associated with altered sebum level is a skin condition associated with elevated sebum levels. According to some embodiments, a skin condition associated with elevated sebum levels is acne.

According to some embodiments, the FABAC of the compositions of the invention comprises two fatty acid radicals wherein at each occurrence W is independently a fatty acid radical having 6-22 carbon atoms; and X is independently a bonding member comprising a heteroatom or a direct C-C or C=C bond. Each possibility represents a separate embodiment of the present invention.

According to some embodiments, the bonding member of said FABAC is selected from the group consisting of: NH, P, S, O, or a direct C-C or C=C bond. Each possibility represents a separate embodiment of the present invention. According to an exemplary embodiment, said bonding member is NH.

According to another embodiment, said one or two fatty acid radicals are independently selected from radicals of a fatty acid selected from the group consisting of: arachidylic acid, stearic acid, behenic acid, palmitic acid, arachidonic acid, eicosapentaenoic acid and oleic acid. Each possibility represents a separate embodiment of the present invention. According to yet another embodiment, said one or two fatty acid radicals is a radical of stearic acid. According to yet another embodiment, said one or two fatty acid radicals is a radical of arachidylic acid.

According to another embodiment, said bile acid is selected from the group consisting of: cholic acid, ursodeoxycholic acid, chenodeoxycholic acid, deoxycholic acid, lithocholic acid, and derivatives thereof. Each possibility represents a separate embodiment of the present invention. According to yet another embodiment, said bile acid is cholic acid.

According to some embodiments, the term "bile salt radical" as used herein refers to a bile salt radical of a bile acid selected from the group consisting of: cholic acid, ursodeoxycholic acid, chenodeoxycholic acid, deoxycholic acid, lithocholic acid, and derivatives thereof. Each possibility represents a separate embodiment of the present invention. According to some embodiments, the bile salt radical is a bile salt radical of cholic acid.

According to some embodiments, said FABAC is selected from the group consisting of:
3-beta-stearoyl-amido,7α,12α-dihydroxy-5-beta-cholan-24-oic acid;
3 -beta arachidylamido,7α,12α-dihydroxy-5-beta-cholan-24-oic acid; and a combination thereof. Each possibility represents a separate embodiment of the present invention.

According to an exemplary embodiment, said FABAC is 3-beta arachidylamido,7α,12α-dihydroxy-5-beta-cholan-24-oic acid (also referred to herein as "Aramchol")..

According to some embodiments, the composition of the invention is formulated for topical application. According to some embodiments, the composition of the invention is a pharmaceutical composition formulated for topical administration. According to other embodiments, the composition of the invention is a pharmaceutical composition formulated for oral administration. According to some embodiments, the pharmaceutical composition comprises at least one pharmaceutically acceptable diluent, carrier or excipient. According to some embodiments, the at least one pharmaceutically acceptable diluent, carrier or excipient is suitable for topical administration.

According to another embodiment, the composition is a topical composition formulated in a form selected from the group consisting of: aqueous solution, cream, lotion, water in oil or oil in water emulsion, multiple emulsion, silicone emulsion, microemulsion, nanoemulsion, gel and an aqueous solution with a co-solvent. Each possibility represents a separate embodiment of the present invention.

Other objects, features and advantages of the present invention will become clear from the following description and drawings.

### BRIEF DESCRIPTION OF THE FIGURES

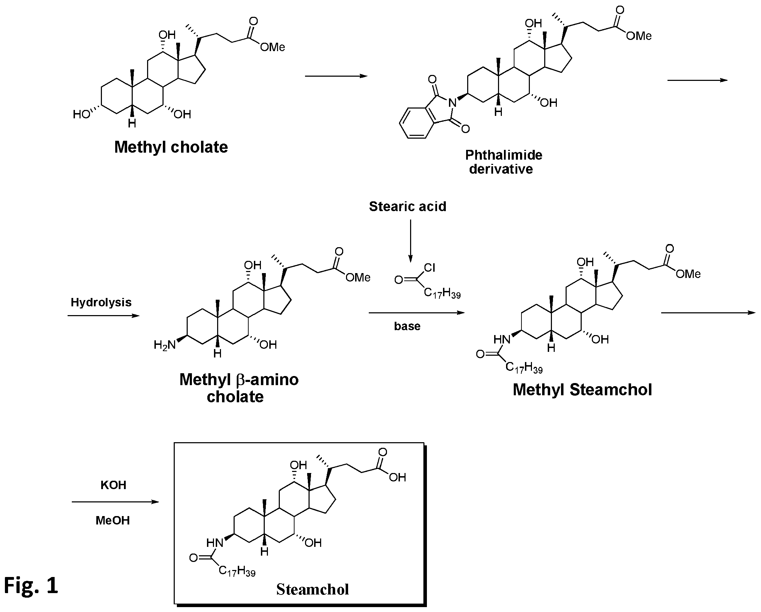

**Figure 1** shows a process for producing 3β-stearylamido-7α,12α, dihidroxy-5β-cholan-24-oic acid (stearyl amido cholanoic acid also referred herein as "Steamchol"), according to some embodiments.
**Figure 2** depicts comparison of viability of epidermal cell cultures which were untreated (Negative Control), treated with 1% Triton X-100 (Positive Control), with DMSO alone, or with DMSO containing either 0.01%, 0.1%, 1% or 2% of Steamchol.

### DETAILED DESCRIPTION OF THE INVENTION

The present invention relates to a bile acid fatty acid conjugates (FABAC), useful in treatment of skin conditions associated with altered sebum levels including but not limited to acne.

The term "FABAC" (synonym BAFAC) as used herein, refers to conjugates of the formula W - X - G (Formula I), wherein G represents a bile acid or bile salt radical thereof, W represents one or two fatty acid radical(s) having 6-22 carbon atoms, and X represents a bonding member between said bile acid and the fatty acid radical(s). According to some embodiments, bonding member X includes, but is not limited to, NH, P, S, O or a direct C=C or C-C bond. Each possibility represents a separate embodiment of the present invention. FABACs are known in the art, and are described, for example, in US Patents 6,384,024, 6,395,722, and 6,589,946, the contents of which are incorporated herein by reference. According to some embodiments, the fatty acid radical(s) comprise specify 8-22 carbon atoms, 14-22 carbon atoms or 18-22 carbon atoms . Each possibility represents a separate embodiment of the present invention. As used herein, the terms "FABACs", "BAFACs", "the FABACs" and "the FABACs of the invention" are used interchangeably. According to some embodiments, the composition of the invention comprises at least one FABAC as an active ingredient.

A non-limiting general structure of FABACs is set forth below. According to a non-liming example, bile acid is conjugated (e.g. using an amide bond, for example at position 3) with 1-2 fatty acids of any of a number of chain lengths.

According to an exemplary embodiment, the FABAC of the invention is 3β-arachidylamido-7α,12α, dihidroxy-5β-cholan-24-oic acid (Arachidyl Amido Cholanoic Acid; an amide conjugate of cholic acid with arachidic acid; also known as "Aramchol" or "C20 FABAC") or 3β-stearylamido-7α,12α, dihidroxy-5β-cholan-24-oic acid (Stearyl Amido Cholanoic Acid; an amide conjugate of cholic acid with stearic acid; also known as "Steamchol" or "C18 FABAC"). Each possibility represents a separate embodiment of the present invention. According to some embodiment, the FABAC is Aramchol.

In another embodiment, the FABAC of compositions of the present invention has the formula I:

W-X-G (I)

wherein G represents a bile acid; W represents one or two saturated or unsaturated fatty acids having 6-22 carbon atoms; and X represents a bonding member or a direct C-C or a C=C bond. According to some embodiments, G represents a radical of a bile acid. According to some embodiments, X represents a bonding member selected from the group consisting of: a heteroatom, a direct C-C bond and a C=C bond. Each possibility represents a separate embodiment of the present invention.

According to some embodiments, the FABACs of compositions of the present invention have the formula II:

(W -X -)n G (II)

wherein G represents a bile acid or a radical thereof; W represents a fatty acid radical having 6-22 carbon atoms; X represents a bonding member comprising a heteroatom or a direct C-C or C=C bond; and n is an integer 1 or 2. Each possibility represents a separate embodiment of the present invention. According to some embodiments, the heteroatom is selected from the group consisting of: NH, P, S and O. Each possibility represents a separate embodiment of the present invention. In general, the term "heteroatom" includes atoms of any element other than carbon or hydrogen, preferred examples of which include nitrogen, oxygen, sulfur, and phosphorus.

According to one embodiment n is 1. According to another embodiment n is 2, and at each occurrence W is independently a fatty acid radical having 6-22 carbon atoms and X is independently a bonding member comprising a heteroatom or a direct C-C or C=C bond. Each possibility represents a separate embodiment of the present invention.

In another embodiment, the bonding member of the FABAC is selected from the group consisting of NH, P, S, O, or a direct C-C or C=C bond. Each possibility represents a separate embodiment of the present invention. According to some embodiments, the term "Direct bond" refers, to a C-C (single) bond. In another embodiment, the term "Direct bond" refers to a C=C (double) bond. In another embodiment, more than one direct bond is utilized in the FABAC of the invention. In another embodiment, the bond between the bile acid and the fatty acid radical(s) is in the beta configuration. In another embodiment, the bond between the bile acid and the fatty acid radical(s) is in the alpha configuration. In another embodiment, the bonding member is other than an ester bond.

According to some embodiments, the bile acid or bile acid radical of the FABAC is selected from the group consisting of: cholic acid, ursodeoxycholic acid, chenodeoxycholic acid, deoxycholic acid, lithocholic acid and derivatives thereof. Each type of bile acid or radical thereof represents a separate embodiment of the present invention. The term "radical" as used herein means a chemical moiety comprising one or more unpaired electrons. According to some embodiments, the bile acid or bile acid radical of the FABAC is cholic acid.

In another embodiment, the FABAC comprises a single fatty acid radical. The conjugation of the bile acid with the fatty acid radical may take place at various positions of the bile acid. In certain embodiments, the conjugation of the bile acid with the fatty acid radical is performed in a position of the bile acid nucleus selected from positions 3, 6, 7, 12 and 24. Each possibility represents a separate embodiment of the present invention. In one embodiment, said conjugation is performed in position 3 of the bile acid nucleus.

In another embodiment, the FABAC comprises two fatty acid radicals. According to some embodiments, the conjugation of each fatty acid radical to the bile acid nucleus is at two positions selected from the 3, 7, 12 and 24 positions of the bile acid nucleus. Each possibility represents a separate embodiment of the present invention. According to a particular embodiment, the conjugations are at position 3 and 7 of the bile acid nucleus.

In another embodiment, the fatty acid is saturated. In another embodiment, the fatty acid is unsaturated. In another embodiment, the fatty acid is mono-unsaturated. In another embodiment, the fatty acid is poly-unsaturated.

In another embodiment, the fatty acid(s) or fatty acid radical(s) of the FABAC are independently selected from the group consisting of: behenic acid, arachidylic acid, stearic acid, and palmitic acid. Each possibility represents a separate embodiment of the present invention.

An exemplary embodiment of a FABAC according to the present invention is presented in Formula III herein below. According to some embodiments, in Formula III n=20 or n=18. Each possibility represents a separate embodiment of the present invention.

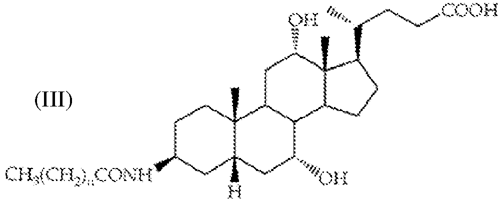

According to some embodiments, the one or two fatty acids or fatty acid radicals of the FABACs of the invention are unsaturated fatty acids or fatty acid radicals. Each possibility represents a separate embodiment of the present invention. In another embodiment, the unsaturated fatty acid(s) or unsaturated fatty acid radical(s) of the FABAC are independently selected from the group consisting of: linolenic acid, eicosapentaenoic acid, docosahexaenoic acid, arachidonic acid, palmipoleic acid, oleic acid and elaidic acid. Each possibility represents a separate embodiment of the present invention. A non-limiting example of FABAC comprising an unsaturated fatty acid is 3β-oleylamido-7α,12α-dihidroxy-5β-cholan-24-oic acid, as depicted in Formula IV herein below.

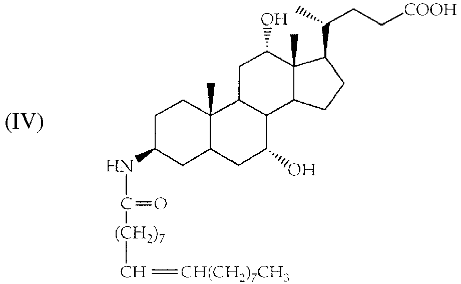

In another embodiment, linoleic acid is utilized. In another embodiment, a conjugated linoleic acid is utilized. In another embodiment, a conjugated linoleic acid isomer is utilized. Each possibility represents a separate embodiment of the present invention. The term "conjugated fatty acid", also known as "CFA", refers to polyunsaturated fatty acids in which at least one pair of double bonds are separated by only one single bond.

In another embodiment, the fatty acid is a short-chain fatty acid. In another embodiment, the fatty acid chain length is 6-8 carbons. In another embodiment, the fatty acid is a medium chain fatty acid. In another embodiment, the fatty acid chain length is 8-14 carbons. In another embodiment, the fatty acid chain length is 14-22 carbons. In another embodiment, the fatty acid chain length is 16-22 carbons. In another embodiment, any other fatty acid chain length known in the art is utilized. Each type of fatty acid or fatty acid radical represents a separate embodiment of the present invention.

According to some embodiments, the FABAC of methods and compositions of the present invention is selected from the group consisting of:
3β-behenylamido-7α,12α-dihydroxy-5β-cholan-24-oic acid; 3β-arachidylamido-7α,12α-dihydroxy-5β-cholan-24-oic acid; 3β-stearylamido-7α,12α -dihydroxy-5β-cholan-24-oic acid; 3β-palmitylamido-7α,12α-dihydroxy-5β-cholan-24-oic acid; 3β-myristylamido-7α,12α-dihydroxy-5β-cholan-24-oic acid; and N-(-carboxymethyl)-3β-stearylamido-7α, 12α-dihydroxy-5β-cholane-24-amide. Each possibility represents a separate embodiment of the present invention.

According to one embodiment, the FABAC of methods and compositions of the present invention is 3β-arachidylamido-7α,12α, dihidroxy-5β-cholan-24-oic acid ("Aramchol"). According to another embodiment, the FABAC of methods and compositions of the present invention is 3β-stearoylamido-7α,12α-dihydroxy-5β-cholan-24-oic acid ("Steamchol").

According to some embodiments, the FABAC of methods and compositions of the present invention is selected from the group consisting of:
3-beta-stearoyl-amido,7α,12α-dihydroxy-5-beta-cholan-24-oic acid;
3 -beta arachidylamido,7α,12α-dihydroxy-5-beta-cholan-24-oic acid;
3 -beta arachidonylamido,7α,12α-dihydroxy-5-beta-cholan-24-oic acid; and a combination thereof. Each possibility represents a separate embodiment of the present invention.

According to some embodiments, the present invention provides pharmaceutical compositions for use in treating skin conditions associated with altered sebum levels. According to certain embodiments, the present invention provides topical compositions for use in treating skin conditions associated with altered sebum levels.

According to some embodiments, altered sebum levels refers to increased sebum levels. According to other embodiments, the present invention provides a pharmaceutical composition formulated for oral administration for use in treating skin conditions associated with increased sebum levels comprising at least one FABAC as the active ingredient. According to some embodiments, the present invention provides topical pharmaceutical compositions for use in treating skin conditions associated with increased sebum levels comprising at least one FABAC as the active ingredient.

According to some embodiments, the present disclosure provides a method of treating skin conditions which may benefit from attenuation, reduction or slowing of keratinocyte differentiation. Each possibility represents a separate embodiment of the present invention. A non-limiting example of a skin condition which may benefit from attenuation, reduction or slowing of keratinocyte differentiation is acne vulgaris.

According to one aspect, the present disclosure provides a method of treating a skin condition associated with altered sebum levels, preferably increased sebum levels, the method comprises the step of administering to a subject in need thereof an effective amount of a composition comprising as an active ingredient a fatty acid bile acid conjugate (FABAC) and a pharmaceutically acceptable diluent or carrier, wherein the FABAC has the formula I:

W-X-G (I)

wherein G represents a bile acid or a bile salt radical thereof; W represents one or two fatty acid radicals having 6-22 carbon atoms; and X represents a bonding member comprising a heteroatom or a direct C-C or C=C bond. Each possibility represents a separate embodiment of the present disclosure.

According to some embodiments, the pharmaceutical composition is formulated for oral administration. According to other embodiments, the pharmaceutical composition is formulated as a topical composition.

According to some embodiments, the present disclosure provides a method of treating a skin condition associated with altered sebum levels, preferably increased sebum levels, the method comprises the step of orally administering to a subject in need thereof an effective amount of a composition comprising as an active ingredient a fatty acid bile acid conjugate (FABAC) and a pharmaceutically acceptable diluent or carrier, wherein the FABAC has the formula I:

W-X-G (I)

wherein G represents a bile acid or a bile salt radical thereof; W represents one or two fatty acid radicals having 6-22 carbon atoms; and X represents a bonding member comprising a heteroatom or a direct C-C or C=C bond. Each possibility represents a separate embodiment of the present disclosure.

According to some embodiments, the present disclosure provides a method of treating a skin condition associated with altered sebum levels, preferably increased sebum levels, the method comprises the step of orally administering to a subject in need thereof an effective amount of a composition comprising Aramchol as an active ingredient. According to some embodiments, the present disclosure
provides a method of treating a skin condition associated with altered sebum levels, preferably increased sebum levels, the method comprises the step of topically administering to a subject in need thereof an effective amount of a composition comprising Steamchol as an active ingredient.

According to some embodiments, the present invention provides a composition for use in treating a skin condition associated with altered sebum levels, preferably increased sebum levels, wherein the composition comprises at least one fatty acid bile acid conjugate (FABAC) as an active ingredient, wherein the FABAC has the formula I:

W-X-G (I)

wherein G represents a bile acid or a bile salt radical thereof; W represents one or two fatty acid radicals having 6-22 carbon atoms; and X represents a bonding member comprising a heteroatom or a direct C-C or C=C bond. Each possibility represents a separate embodiment of the present invention. According to some embodiments, the composition is a pharmaceutical composition formulated for oral administration and further comprises at least one carrier, diluent or excipient suitable for oral administration.

According to some embodiments, the present disclosure provides a topical pharmaceutical composition, wherein the composition comprises at least one fatty acid bile acid conjugate (FABAC) as an active ingredient, wherein the FABAC has the formula I:

W-X-G (I)

wherein G represents a bile acid or a bile salt radical thereof; W represents one or two fatty acid radicals having 6-22 carbon atoms; and X represents a bonding member comprising a heteroatom or a direct C-C or C=C bond; and wherein the pharmaceutical composition is formulated for topical administration and further comprises at least one pharmaceutically acceptable diluent or carrier. Each possibility represents a separate embodiment of the present invention.

According to some embodiments, the present disclosure provides use of a pharmaceutical composition comprising at least one FABAC for preparation of a medicament for treatment of a skin condition associated with altered sebum levels, preferably increased sebum levels. Each possibility represents a separate embodiment of the present invention. According to some embodiments, the present invention provides use of a pharmaceutical composition comprising Aramchol for preparation of a medicament for treatment of a skin condition associated with altered sebum levels, preferably increased sebum levels. Each possibility represents a separate embodiment of the present invention.

According to some embodiments, the present disclosure provides use of a topical pharmaceutical composition comprising at least one FABAC for preparation of a medicament for treatment of a skin condition associated with altered sebum levels, preferably increased sebum levels. Each possibility represents a separate embodiment of the present invention. According to some embodiments, the present invention provides use of a topical pharmaceutical composition comprising Steamchol for preparation of a medicament for treatment of a skin condition associated with altered sebum levels, preferably increased sebum levels. Each possibility represents a separate embodiment of the present disclosure.

According to some embodiments, the present disclosure provides a pharmaceutical composition comprising at least one FABAC of the invention as an active ingredient, wherein the pharmaceutical composition is formulated for topical administration. According to some embodiments, the present disclosure provides a pharmaceutical composition comprising Aramchol as an active ingredient, wherein the pharmaceutical composition is formulated for topical administration. According to some embodiments, the present disclosure provides a pharmaceutical composition comprising Steamchol as an active ingredient, wherein the pharmaceutical composition is formulated for topical administration. The pharmaceutical composition of the invention is provided for treatment of a condition associated with altered sebum level, preferably increased sebum level. Each possibility represents a separate embodiment of the present invention. According to some embodiments, the present disclosure provides a method of treating a condition associated with altered sebum level by orally or topically administering a pharmaceutical composition comprising at least one FABAC selected from Aramchol and Steamchol as an active ingredient. Each possibility represents a separate embodiment of the present disclosure.

The term "skin conditions associated with altered sebum levels" as used herein includes but is not limited to sebum over-production conditions. The term "sebum over-production condition" as used herein includes without limitation a condition in which a large amount of sebum is produced by the skin of a subject, thus resulting in a pathological condition or undesirable condition. Examples of such sebum overproduction conditions include, but are not limited to, acne, seborrhea, seborrhoeic dermatitis, a sebaceous cyst and sebaceous hyperplasia and related conditions. Each possibility represents a separate embodiment of the present invention. According to some embodiments, a sebum overproduction condition is acne. According to some embodiments, acne refers to Acne Vulgaris. It is to be noted that although acne is a skin condition associated with altered or over-produced sebum level, the compositions of the invention may be suitable for treatment of acne which is not manifested by over-production or excess secretion of sebum.

According to some embodiments, the condition associated with altered sebum levels is selected from the group consisting of: acne, seborrhea, seborrhoeic dermatitis, a sebaceous cyst, sebaceous hyperplasia and related conditions and a combination thereof. Each possibility represents a separate embodiment of the present invention. According to some embodiments, the condition associated with altered sebum levels is acne. According to some embodiments, acne refers to severe acne. According to some embodiments, severe acne is cystic acne. According to some embodiments, the condition associated with altered sebum levels is other than atopic dermatitis.

According to some embodiments, the acne is selected from the group consisting of: acne vulgaris, cystic acne, acne atrophica, bromide acne, chlorine acne, acne conglobata, acne cosmetica, acne detergicans, epidemic acne, acne estivalis, acne fulminans, halogen acne, acne indurata, iodide acne, acne keloid, acne mechanica, acne papulosa, pomade acne, premenstral acne, acne pustulosa, acne rosacea, acne scorbutica, acne scrofulosorum, acne urticata, acne varioliformis, acne venenata, propionic acne, acne excoriee, gram negative acne, steroid acne, nodulocystic acne and a combination thereof. Each possibility represents a separate embodiment of the present invention.

"Skin", as used herein, refers to any epidermal surface prone to acne and can also include, without limitation, the surface of the face and neck, hands, elbows, upper arm region, knees, thighs, legs, feet, breasts, chest, stomach, buttocks, and back area. Each possibility represents a separate embodiment of the present invention. According to a preferable embodiment, the term "skin" refers to the surface of the face and neck.

In some embodiments, the method of treatment according to the present disclosure results in at least one of: a reduction of sebum production, a reduction in hyperkeratinization, a reduction in colonization by P. acnes, a reduction in release of inflammatory mediators into the skin and a combination thereof. Each possibility represents a separate embodiment of the present disclosure. In some embodiments, the disclosed method of treatment is effective to reduce a symptom of skin inflammation including, for example, redness, swelling, leukocyte infiltration, and/or lesion development. Each possibility represents a separate embodiment of the present disclosure.

In some embodiments, the disclosed method of treatment is effective to reduce the severity of a skin condition associated with acne including, for example, scaling, erythema, itching, burning, and/or stinging. Each possibility represents a separate embodiment of the present disclosure.

In some embodiments, the disclosed method of treatment is effective to reduce at least one of the size, redness and itchiness of acne lesions. Each possibility represents a separate embodiment of the present disclosure.

In some embodiments, the disclosed method of treatment results in a delay in recurrence of an acute acne outbreak in the subject.

In some embodiments, the disclosed method of treatment results in a reduction in the severity of a recurring acute acne outbreak in the subject.

In some embodiments, the disclosed method of treatment is effective in treating a condition associated with altered sebum levels, such as, but not limited to, acne at least by reducing expression level of keratin 10 (KRT 10) and/or keratin 1 (KRT 1) in skin cells. Each possibility represents a separate embodiment of the present disclosure.

In some embodiments, the disclosed method of treatment is effective in treating a condition associated with altered sebum levels, such as, but not limited to, acne at least by increasing cholesterol efflux in skin cells.

In some embodiments, administration of the disclosed composition according to the method of the disclosure results in little to no effect on viability of skin cells. In some embodiments, topical administration of the disclosed composition to the skin of a subject according to the method of the disclosure results in little to no effect on viability of skin cells. According to some embodiments, administration of the disclosed composition to a subject results in less than 5%, preferably less than 4% change in viability of cells in the skin following administration. Each possibility represents a separate embodiment of the present disclosure.

As used herein, the terms "effective amount" or "therapeutically effective amount" refer to an amount of a composition that is capable of inhibiting, reducing, attenuating or treating at least part of the symptoms of the skin condition associated with altered sebum levels (e.g., acne). According to some embodiments, a dermatologically effective amount of a composition relates to an amount sufficient to inhibit and/or treat and/or attenuate and/or reduce at least part of the symptoms of the skin condition associated with altered sebum levels upon topical or oral administration of the composition to the subject's skin. Each possibility represents a separate embodiment of the present invention. For example, a therapeutically effective amount of a composition may be a composition comprising at least one FABAC as an active agent that improves or treats the symptoms of acne such as acne vulgaris.

The specific dose of a composition administered according to the present invention will, of course, be determined by particular circumstances such as, but not limited to, the particular FABACs in the composition administered, the mode of administration, the physiological state of the subject and the severity of the pathological condition being treated. Therapeutically effective amounts, or doses, appropriate for an individual subject may be readily determined using routine clinical techniques well known to those skilled in the art (such as dose response plots). According to some embodiments, the disclosed composition is typically administered in several dosages over a prolonged time period until a sufficient response has been achieved. According to some embodiments, a sufficient response is inhibition and/or treatment and/or amelioration of at least part of the symptoms of the skin condition associated with altered sebum levels.

According to some embodiments, one of ordinary skill in the art may determine an effective amount of the disclosed composition by methods such as, but not limited to, histology, staining skin samples using Hematoxylin and Eosin (H&E), immunostaining skin samples for keratin 1 and/or keratin 10 levels, observing abscess formation and combinations thereof. According to some embodiment, an effective amount of the disclosed composition is an amount sufficient to reduce expression of keratin 1 and/or keratin 10 in the subject's skin. Each possibility represents a separate embodiment of the present invention. According to some embodiment, an effective amount of the disclosed composition is an amount sufficient to induce at least a 2-fold reduction in the expression level of keratin 1 and/or keratin 10 in the subject's skin. Each possibility represents a separate embodiment of the present invention. Without wishing to be bound by any theory or mechanism, administering the disclosed composition to a subject afflicted with a condition associated with elevated sebum levels may result in at least 2-fold decrease in expression of keratin 1 and/or 10 in the skin of the subject, thus treating said condition.

One of skill in the art may also confirm that an effective amount of a composition has been administered to a subject with a condition associated with altered sebum levels by simply observing or measuring the change in an area affected by the condition before treatment and a reasonable time after treatment. According to some embodiments, the compositions of the disclosure comprise therapeutically effective amounts of the active components, such as at least one FABAC.

According to some embodiments, the methods of the disclosure comprise administering a therapeutically effective amount of the active agent (such as a FABAC), or a pharmaceutical composition containing it to a subject in need thereof. Each possibility represents a separate embodiment of the present disclosure.

According to some embodiments, the pharmaceutical composition of the invention is formulated for topical administration. According to some embodiments, the composition is formulated for topical administration in a form such as, but not limited to, a solution, ointment, gel, cream, foam or any form for local application (such as subcutaneous injection). Each possibility represents a separate embodiment of the present invention. According to some embodiments, the pharmaceutical composition may be topically administered by way of a drug eluting device, such as gauze, a patch, pad, or a sponge. Each possibility represents a separate embodiment of the present invention. According to some embodiments, the active agent of the disclosed composition is at least one FABAC. According to some embodiments, the active agent of the disclosed composition is Aramchol.

According to some embodiments, the frequency and duration of administration in order to treat a skin condition associated with altered sebum levels in a subject, as indicated, are those sufficient to achieve the desired treatment endpoint, such as, but not limited to, complete remission of acne. One of ordinary skill in the art can readily determine a suitable course of treatment utilizing the compositions and methods according to the present disclosure.

According to some embodiments, the composition comprises pharmaceutically acceptable or cosmetically acceptable media, excipients, carrier or additives. Each possibility represents a separate embodiment of the present invention. According to some embodiments, the pharmaceutically acceptable or cosmetically acceptable media, excipients, carrier or additives are suitable for topical administration. According to some embodiments, the pharmaceutically acceptable or cosmetically acceptable media, excipients, carrier or additives are suitable for oral administration. According to some embodiments, the pharmaceutically acceptable or cosmetically acceptable media, excipients, carrier or additives enhance follicular delivery and/or deposition. Each possbibility represents a separate embodiment of the present invention. According to some embodiments, pharmaceutically acceptable or cosmetically acceptable media, excipients, carrier or additives do not pose a risk of toxicity, intolerance, instability, or allergic reaction to the skin.

According to some embodiments, the pharmaceutically acceptable or cosmetically acceptable media, excipients, carrier or additives do not interact with the FABACs in a way which reduces the efficacy of the composition in treatment of a condition associated with altered sebum levels.

According to some embodiments, the present disclosure provides a method of treating acne, preferably acne vulgaris, in a subject in need thereof, the method comprising administering a pharmaceutical composition comprising at least one FABAC as an active ingredient to said subject. According to some embodiments, the present disclosure provides a method of treating acne, preferably acne vulgaris, in a subject in need thereof, the method comprising administering a pharmaceutical composition comprising at least one FABAC selected from Aramchol and Steamchol as an active ingredient to said subject. Each possibility represents a separate embodiment of the present disclosure.

According to some embodiments, a subject in need thereof refers to a subject afflicted with acne. According to some embodiments, administering is administering orally. According to some embodiments, administering is administering topically to the skin of the subject.

According to some embodiments, the present invention provides a pharmaceutical composition formulated for topical or oral administration for treatment of a condition associated with increased sebum level, preferably acne, most preferably acne vulgaris, the composition comprising at least one FABAC as an active ingredient, preferably Aramchol. Each possibility represents a separate embodiment of the present invention.

An early sign of acne may be a plug which is produced by hair, sebum and keratinocytes which fill the hair follicle. The plug prevents sebum from reaching the surface of the skin through a pore. The mixture of oil and cells may allow the most common anaerobes living on the skin, Propionibacterium acnes (P. acnes), to grow in the plugged follicle. These bacteria produce chemicals and enzymes and attract white blood cells that cause inflammation (e.g., swelling, redness, heat or pain). Propionibacterium acnes is associated with acne vulgaris and linked to certain cases of endocarditis, anaerobic arthritis, wound infections and abscesses.

A small number of acne patients with severe disease show little or no response to intensive therapeutic efforts including the use of high doses of oral tetracycline, dapsone, prednisone, and, in women, estrogen. In many cases, these drugs afford only a modest degree of control while the side effects of these agents severely restrict their usefulness. Patients with nodulocystic acne suffer from large, inflammatory, suppurative nodules appearing on the face, and frequently the back and chest. In addition to their appearance, the lesions are tender and often purulently exudative and hemorrhagic. Disfiguring scars are frequently inevitable.

Known therapies for acne often involve both local and systemic administration of vitamin A derived compounds, collectively known as retinoids. Topical application of all-trans-retinoic acid has been tried with some success, particularly against comedones or blackheads, but this condition frequently returns when the treatment is withdrawn. Additionally, retinoic acid applied topically can be highly irritating and its use can be painful for the patient depending on the concentration used and the frequency of application.

A number of side effects complicate the administration of large doses of vitamin A derivatives. Among the many- symptoms of hypervitaminosis A are weight loss, desquamation of the skin, hair loss, irritation of the oral and pharyngeal mucosa, and nose bleeds, headaches, bone pain, liver toxicity due to storage of vitamin A in the liver, papilledena, pseudotumor cerebri, demineralization, and periosteal thickening of the bones. Because of these and other side effects of oral treatment with vitamin A derivatives and all- trans-retinoic acid, which produces similar side effects, they are rarely recommended for dermatopathic conditions.

Furthermore, retinoids have been shown to be cytotoxic for fibroblasts and epithelial cells in the range of 0.6-3*10⁵ M (Varani et al. Journal of Investigative Dermatology (1993) 101, 839-842), and to increase epithelial cell death (Ding et al. Invest Ophthalmol Vis Sci. 2013 Jun 26;54(6):4341-50).

Isotretinoin first marketed as Accutane (Roaccutane) by Hoffmann-La Roche, is a medication used mostly for cystic acne. Due to side-effects (advanced bone age and premature epiphyseal closure (stunted growth), decreased night vision, hyperostosis, inflammatory bowel disease, keloids , keratoconjunctivitis sicca (dry eyes), xeroderma (dry skin), teratogenicity (birth defects), depression, changes in brain) and high cost, isotretinoin is used primarily for severe cystic acne and acne that has not responded to other treatments. Acne treatment usually begins with topical medications (e.g., benzoyl peroxide and adapalene), followed by oral antibiotics and isotretinoin as a last resort. Despite being in use for a long time, the exact mechanism of action in these compounds is not fully elucidated. The general hypothesis is that retinoids normalize keratinocytes differentiation. Other possible mechanisms of retinoids could include down regulation of desmosomal proteins, an anti-proliferative effect, regulation of lipid synthesis, growth factors and cytokines.

Retinoids are thought to exert their multi- faceted array of effects through their receptors which are nuclear receptors. There are at least 6 retinoic acid receptors belonging to two families RARs and RXRs. Nuclear receptors expressed in keratinocytes include the retionid receptors RARs and RXRs, vitamin D3 receptor and thyroid hormone receptor, all can affect keratinocytes differentiation. RAR gamma receptor for retinoic acid plays an important role in the morphogenesis and differentiation of squamous epithelia. Both RAR alpha and RAR gamma are present in skin with RAR gamma being the predominant specie.

Retinoic acid was shown to inhibit the activity of keratinocytes transgultaminase and the formation of the cornified envelope. Interestingly, not all differentiation markers are regulated by retinoic acid. Involucrin for example, is unaffected when cultured keratinocytes are treated by retinoic acid. Retinoic acid regulates the differentiation and proliferation of epidermal keratinocytes by acting as an antagonist of activating protein 1 (AP-1). AP-1 consists of combinations of fos and jun heterodimers, which are well-established regulators of keratinocytes differentiation. Both keratins 1 and 10 were shown to be down regulated when skin is treated with retinoic acid both in vitro and in vivo.

Without wishing to be bound by any theory or mechanism, the fatty acid-bile acid conjugates (FABAC's) exhibit "retinoid like" mode of action not via activation of nuclear receptors but via an effect of downstream biochemical cascades, as suggested by the results of the gene expression study exemplified herein below.

According to some embodiments, administration of FABACs or a composition comprising same results in at least one effect selected from the group consisting of: facilitation of ABCA1 activity in skin cells, inhibition of Stearoyl CoA- Desaturase -1 (SCD1) activity in skin cells, down-regulation of keratin 1 and/or 10 expression in skin cells and any combination thereof. Each possibility represents a separate embodiment of the present disclosure.

According to some embodiments, down-regulation of kertain 1 and/or 10 expression is indicative of down-regulation of keratinocyte differentiation. Without wishing to be bound by any theory or mechanism, down-regulation of keratinocytes differentiation by FABCAs as indicated by down regulation of keratin 1 and/or 10 provides an advantageous effect in preventing clogging of follicular opening, thus contributing to treatment of conditions of elevated sebum levels, such as, but not limited to, acne.

SCD-1 is an enzyme that catalyzes the synthesis of monounsaturated fatty acids from saturated fatty acids. SCD1 is expressed in liver, adipose tissue, skeletal muscle and skin. Experiments with mice deficient in SCD1 and SCD1 inhibition demonstrated the following effects on skin: Sebaceous gland hypoplasia (in SCD1 deficient mice), depletion in sebaceous lipids, dry skin and alopecia. Researchers note that the reduction in sebaceous lipids upon deletion of SCD1 may be of value in the treatment of disease such as acne vulgaris which is associated with increased sebaceous activity. SCD1 deletion, however, has been additionally shown to be associated with hyperkeratosis and inflammation (Sampath et al., J. Biol. Chem. 2009, 284:19961-19973).

Interestingly, in skin, the inhibition of SCD1 activity did not affect the strength of the barrier. This may be a result of compensation mechanism. While inhibition of SCD1 leads to increase in ABCA1 transporter activity and depletion in barrier cholesterol levels in keratinocytes, a compensatory enhanced production of cholesterol and ceramides may lead to a replenished barrier.

The cause for sebocytes atrophy may further be associated with decrease in ligands activating peroxisome proliferator-activated receptor (PPAR). Similar to other nuclear hormone receptors, PPARs act as ligand-activated transcription factors. When bound to its fatty acid ligand, PPARα forms a heterodimeric complex with the retinoid X receptor (RXR) to regulate transcription. PPARγ is activated by the inflammatory mediators prostaglandins and leukotrienes and regulates the gene expression of proteins involved in the storage of fatty acids. PPARβ is weakly activated by fatty acids, prostaglandins, and leukotrienes (Fajas, L., et al, 2001). Decrease in PPAR activation ligands may lead to:
- Activation of ABCA1 transporter.
- Decreased retinol esterification.

Table 1 herein below summarizes key physiological conditions leading to acne. Without wishing to be bound by any theory or mechanism of action, table 1 further summarizes the activity of fatty acid-bile acid conjugates (FABACs) in treating acne, according to some embodiments, based on their structure and the experimental data generated from a gene expression study, as exemplified herein below.

**Table 1. Conditions leading to acne and anticipated effect of FABACs**

| **Abnormality in acne** | **Clinical/ Physiological effect** | **Potential FABAC activity to attenuate the condition** |
|---|---|---|
| Increased sebum production as a result of elevated blood hormone levels or other abnormal physiology | Sebum serves as a nutrient fostering bacterial proliferation inside the gland | SCD1 enzyme inhibition that is associated with: Depletion in sebum lipids and/or Hyppoplasia of the sebaceous gland and reduction in its activity |
| Abnormal microbiota population, and especially propionobacterium acnes (p. acne) | P. acne consumes sebum and over proliferates resulting in release of inflammatory fatty acids and toxins | Due to its chemical properties, lipophilicity and relatively low Mw, FABAC may intercalate into the bacterial wall, create imbalance and partial perturbation of the barrier thereof and therefore may decrease bacterial population |
| Cornification of the pilosebaceous duct that narrows the duct opening and restricts sebum drainage | Sebum does not flow freely from the gland onto the surface therefore appropriate drainage of the gland is not achieved | As exemplified herein, FABAC significantly inhibit the expression of keratins 1 and 10 which are markers for keratinocytes differentiation. The decrease in rate of differentiation can attenuate cornification rate of keratinocytes to corneocytes and provide relief of pore clogging of the pilosebaceous opening, thus allowing more effective sebum drainage |
| Inflammation | Humoral immune response to free fatty acids and toxins; recruitment of inflammatory immune cells and biomarkers that leads to skin damage and can cause scarring | Depletion in sebum lipid and potential anti-microbial activity can lead to reduction in inflammation |

### Formulations

According to some embodiments, the composition of the invention is formulated as a pharmaceutical composition comprising at least one FABAC as an active agent. According to some embodiments, the composition of the invention is formulated for oral administration, comprising at least one FABAC as an active agent and further comprising at least one carrier, diluent or excipient suitable for oral administration.

According to some embodiments, the composition of the invention is formulated as a topical composition comprising at least one FABAC as an active agent. According to some embodiments, the topical composition is formulated for topical administration to the skin of the subject.

The compositions of the present invention may be manufactured by processes well known in the art, e.g., by means of conventional mixing, dissolving, granulating, dragger-making, levigating, emulsifying, encapsulating, entrapping or lyophilizing processes.

The compositions for use in accordance with the present invention thus may be formulated in conventional manner using one or more physiologically acceptable carriers comprising excipients and auxiliaries, which facilitate processing of the active compounds into preparations which can be used pharmaceutically. Proper formulation is dependent upon the route of administration chosen.

According to some embodiments, the topical compositions of the invention comprise a therapeutically, dermatologically or cosmetically acceptable carrier to act as a diluent, dispersant or vehicle for at least one FABAC, so as to facilitate its distribution when the composition is applied to the skin. Vehicles other than, or in addition to, water may include liquid or solid emollients, solvents, humectants, thickeners and powders. Each possibility represents a separate embodiment of the present invention.

According to some embodiments, the composition of the present invention may be formulated for topical administration in the form of aqueous or non-aqueous solutions, lotions, creams, gels, ointments, foam, mousse, sprays, emulsions, microemulsions, adhesive patches, powders etc. Each possibility represents a separate embodiment of the present invention. The formulation may be oleaginous-based, occlusive composition comprising, for example, white petroleum and or mineral oil. In some embodiments the composition is non-greasy or substantially non-greasy and can be a water-based formulation.

According to some embodiments, the present invention provides a therapeutic or dermopharmaceutical composition comprising at least one FABAC or a dermatological or pharmaceutically-acceptable salt or ester thereof, and further comprising a therapeutically, cosmetically or dermatologically acceptable diluent, carrier or excipient. Each possibility represents a separate embodiment of the present invention.

According to some embodiments, the composition of the invention comprises at least one FABAC. According to some embodiments, the FABAC in the composition is in an effective amount to induce treatment and/or inhibition and/or attenuation of at least one symptom of the condition associated with altered sebum levels. As used here, the terms "effective amount" and "effective concentration" are used interchangeably.

According to some embodiments, the effective concentration of the FABAC in the composition is between about 0.005 to 20% weight/volume, optionally between 0.01 to 10% weight/volume, possibly between 0.01 to 5% weight/volume, alternatively between 0.05 to 2% weight/volume. Each possibility represents a separate embodiment of the present invention. According to some embodiments, the weight/volume concentration of FABAC in the composition of the invention is 0.01%-2%, possibly 0.1%-2%, alternatively between 1%-2%. Each possibility represents a separate embodiment of the present invention. According to some embodiments, the weight/volume concentration of FABAC in the composition of the invention is at least 0.01%. According to some embodiments, the FABAC in the composition of the invention is Aramchol. According to some embodiments, the FABAC in the composition of the invention is Steamchol. According to some embodiments, the concentration of the FABAC in the composition is that which provides treatment and/or amelioration and/or prevention of at least one symptom of a condition associated with altered sebum level, preferably with an elevated sebum level. Each possibility represents a separate embodiment of the present invention.

In some embodiments, the composition of the invention further comprises at least one additional active ingredient, other than the FABACs of the invention. In some embodiments, the composition of the invention further comprises at least one additional active ingredient, other than the FABACs of the invention, normally used for cosmetics or external skin treatments. Non-limiting examples of such additional active ingredients include, but are not limited to, the following classes of ingredients: whitening agent, moisture retainers, anti-oxidants, oil ingredients, UV absorbers, surfactants, thickeners, alcohols, powder components, coloring agents, aqueous ingredients, water, various skin nutrients, stabilizing agents, anti-inflammatory agents (non-steroidal and steroidal), anti-viral agent (such as benzoyl peroxide, octopirox, erythromycin, triclosan, azelaic acid and its derivatives, etc.), anti-psoroiatic agents, sebum miscible agents, other anti-acne agents (including desquamators, keratolytics, comedolytics and exfoliants), antibacterials (including antibiotics and antimicrobials), antifungals, anti-dandruff, antiprotozoals, and the like. Each possibility represents a separate embodiment of the present invention.

In some embodiments, which may be used or combined with any of the above or below mentioned embodiments, the antibiotic is selected from the group consisting of: a penicillin, a polyketide antibiotic, a cephalosporin, a lincosamide, a quinolone, a folic acid synthesis inhibitor, a tetracycline, a rifamycin, a sulfonamide, an aminoglycoside, fusidic acid, a polypeptide antibiotic, a lipopeptide antibiotic, chloramphenicol and mupirocin. Each possibility represents a separate embodiment of the present invention.

In some embodiments, which may be used or combined with any of the above or below mentioned embodiments, the antibiotic is an antibiotic suitable for topical use. In some embodiments, which may be used or combined with any of the above or below mentioned embodiments, the antibiotic is an antibiotic suitable for oral use.

In additional embodiments, the at least one additional active ingredient is an ingredient used and/or approved for the treatment of acne. In additional embodiments, the at least one additional active ingredient is an ingredient used and/or approved for the treatment of acne and suitable for topical administration. According to some embodiments, the at least one additional active ingredient is an ingredient suitable for topical administration which is used and/or approved for the treatment of acne including, but not limited to,, benzoyl peroxide, topical retinoids (e.g., tretinoin, adapalene, isotretinoin, tazarotene), and/or topical antibiotics (e.g., clindamycin, erythromycin, tetracycline). Each possibility represents a separate embodiment of the present invention. Such topical treatment may include other topical therapies (e.g., salicylic acid, sulphur, resorcinol, sodium sulfacetamide, aluminium chloride, zinc, nicotinamide, triethyl citrate/ethyl linoleate, dapsone, taurine bromamine, azelaic acid, sodium sulfacetamide 10%/sulfur 5%) as well as combination topicals (e.g., combinations of topical treatments with different mechanisms of action).

According to some embodiments, the disclosed method further comprises administration of an additional active agent used and/or approved for the treatment of acne. According to some embodiments, the additional active agent used and/or approved for the treatment of acne is configured for topical administration. According to some embodiments, the additional active agent used and/or approved for the treatment of acne is configured for oral administration.

In some embodiments, the at least one additional active agent used and/or approved for the treatment of acne and administered as an oral treatment, includes, but is not limited to, oral antibiotics (e.g., tetracyclines such as tetracycline, oxytetracycline, doxycycline, lymecycline or less preferably minocycline; cotrimoxazole; quinolones such as ciprofloxacin; aminoglycosides; chloramphenicol; clindamycin; macrolides such as erythromycin and azitromycin; trimethoprim), oral contraceptives (e.g., combined oral contraceptives that contain an estrogen such as ethinylestradiol and a progestogen; progestogen; estrogen), and/or oral isotretinoin. Each possibility represents a separate embodiment of the present invention.

The compositions of the present invention may be present in the forms of aqueous or hydroalcoholic solution, solubilized systems, emulsions, powders, oils, aqueous or anhydrous gels, serum, ointments, aerosols, water-oil two-phase systems, water-oil-powder three-phase systems, foams etc. Each possibility represents a separate embodiment of the present invention. In some embodiments the composition is applied in a form selected from the group consisting of: a facial cleanser, spray, salve, ointment, lotions, emulsions, creams, gels, essences (beauty lotions), packs patches and masks. Each possibility represents a separate embodiment of the present invention.

In other embodiments, such as in the case of makeup cosmetics, the composition is topical and may be used with a wide range of types of cosmetics such as foundations. In additional embodiments, the topical composition is applied in the form of a toiletry product, e.g., body soap, facial soap, etc. According to some embodiments, the composition may be formulated as a quasi-drug. According to some embodiments, in the case of quasidrugs, the composition can be formulated for a wide range of applications such as various ointments.

The types or forms of the compositions of the present invention are not limited to these forms and types. In any case, the person skilled in the art will ensure that additives, the amounts thereof and the selected formulation are selected so as not to be detrimental to the desired, advantageous properties of the composition according to the invention.

According to some embodiments, the disclosed composition is a topical composition formulated in a form selected from the group consisting of: aqueous solution, cream, lotion, water in oil or oil in water emulsion, multiple emulsion, silicone emulsion, microemulsion, nanoemulsion, gel, serum and milk. Each possibility represents a separate embodiment of the present invention.

Non-limiting examples of suitable topical formulations of the disclosed composition are as follows:

### Lotions and Creams

According to some embodiments, the composition is formulated as a lotion. The lotions contain an effective concentration of one or more FABAC compound as described herein. The compositions of the present invention may also include at least one or more emollient, which can function as either or both a lubricating and thickening agent. The emollients can comprise in total from about 0.1% to about 50%, preferably from about 1% to about 10%, by weight of the composition. According to certain embodiments, the emollients can comprise at least 0.01% by weight of the composition. Any emollients known to those of skill in the art as suitable for application to human skin may be used. These include, but are not limited to: hydrocarbon oils and waxes, including mineral oil, petrolatum, paraffin, ceresin, ozokerite, microcrystalline wax, polyethylene, and perhydrosqualene; silicone oils; triglyceride fats and oils, including those derived from vegetable, animal and marine source; including jojoba oil and shea butter; acetoglyceride esters, such as acetylated monoglycerides; ethoxylated glycerides, such as ethoxylated glyceryl monostearate; fatty acids, fatty alcohols and derivatives thereof. Each possibility represents a separate embodiment of the present invention. Other suitable emollients include lanolin and lanolin derivatives; polyhydric alcohols and poly ether derivatives; polyhydric alcohol esters; wax esters; vegetable waxes; phospholipids, such as lecithin and derivatives; sterols, including, but not limited to, cholesterol and cholesterol fatty acid esters; amides, such as fatty acid amides, ethoxylated fatty acid amides, and solid fatty acid alkanolamides. Each possibility represents a separate embodiment of the present invention.

The lotions may further contain from about 1% to about 10%, more preferably from 2% to 5%, of an emulsifier. Each possibility represents a separate embodiment of the present invention The emulsifiers may be nonionic, anionic, cationic or a mixture thereof. Each possibility represents a separate embodiment of the present invention. Suitable emulsifiers are known to those with skill in the art. Other conventional components of such lotions and creams may be included. One such additive is a thickening agent at a level from 1% to 10% of the composition. Examples of suitable thickening agents include, but are not limited to: cross-linked carboxypolymethylene polymers, ethyl cellulose, polyethylene glycols, gum tragacanth, gum karaya, xanthan gums, bentonite and other clays, hydroxy ethyl cellulose, and hydroxypropyl cellulose. Each possibility represents a separate embodiment of the present invention

According to some embodiments, the lotions and creams are formulated by simply admixing all of the components together. According to some embodiments, the FABAC is dissolved, suspended or otherwise uniformly dispersed in the mixture.

### Solutions and Suspensions

According to some embodiments, the composition is formulated as a solution. According to some embodiments, the composition is formulated as a suspension. According to some embodiments, the solutions, which may be aqueous or non-aqueous, are formulated to contain an effective concentration of one or more FABAC compound as disclosed herein.
Suitable organic materials which may be useful as the solvent or a part of a solvent system in the solution are as follows: propylene glycol, polyethylene glycol, polypropylene glycol, glycerin, sorbitol esters, 1,2,6-hexanetriol, ethanol, isopropanol, diethyl tartrate, butanediol, and mixtures thereof. Each possibility represents a separate embodiment of the present invention. Such solvent systems can also contain water.

According to some embodiments, the composition is formulated as an emulsion. When the compositions of the invention are formulated as an emulsion, the proportion of the fatty phase may range from about 5% to about 80% by weight, and preferably from about 5% to about 50% by weight, relative to the total weight of the composition. Each possibility represents a separate embodiment of the present invention. Oils, emulsifiers and co-emulsifiers incorporated in the composition in emulsion form are selected from among those known to those with skill in the cosmetic or dermatological field.

The compositions formulated as solutions or suspensions may be applied directly to the skin, or, may be formulated as an aerosol and applied to the skin as a spray, foam or mousse. Each possibility represents a separate embodiment of the present invention. The aerosol compositions may further contain from about 20% to 80%, preferably from 30% to 50%, of a suitable propellant. Each possibility represents a separate embodiment of the present invention. Examples of such propellants may be, but are not limited to, the chlorinated, fluorinated and chlorofluorinated lower molecular weight hydrocarbons. Nitrous oxide, carbon dioxide, butane, and propane may also be used as propellant gases. These propellants are used as known in the art in a quantity and under a pressure suitable to expel the contents of the container. In the case of a pressurized aerosol, the dosage unit may be determined by providing a valve to deliver a metered amount.

### Gels and Solids

According to some embodiments, the composition is formulated as a gel. Gel compositions may be formulated by simply admixing a suitable thickening agent with the previously described solution or suspension compositions. Examples of suitable thickening agents have been described herein above with respect to lotions. According to some embodiments, the gelled compositions contain an effective concentration of at least one FABAC compound. According to some embodiments, the composition further comprises from about 5% to about 75% of an organic solvent as previously described; from about 0.5% to about 20% of a thickening agent, and the balance being water or other aqueous carrier.

In other embodiments the compositions formulated as solutions, suspensions lotions and gels of the present invention are formulated as a foam or mousse for dermal application. Each possibility represents a separate embodiment of the present invention. Relevant carriers for formulation as a foam or mousse are taught, for example, in International Patent Application Publication No. WO 2004/037225 and US Patent No. 6,730,288.

According to some embodiments, the composition is formulated as a solid form. According to some embodiments, the composition is formulated as a semi-solid form. According to some embodiments, a semi-solid form is an emulsion or a microemulsion. Compositions of semi-solid forms may be formulated as stick-type compositions intended for application to the lips or other parts of the body. The solids may also contain from about 50% to about 98% of the previously described emollients. This composition may contain from about 0.1% to about 20% w/w, of a suitable thickening agent, and, if desired or needed, emulsifiers and water or buffers. Thickening agents previously described with respect to lotions may be suitably employed in the compositions in solid form. Other ingredients, such as preservatives, including methyl-paraben or ethyl-paraben, perfumes, dyes or the like, that are known in the art to provide desirable stability, fragrance or color, or other desirable properties, may be further added to compositions for topical application to the skin.

### Oral Administration

According to some embodiments, the composition of the invention is formulated for oral administration. According to some embodiments, the composition of the invention is a pharmaceutical composition formulated for oral administration, comprising at least one FABAC of the invention as an active ingredient.

According to some embodiments, oral administration is in the form of hard or soft gelatin capsules, pills, capsules, tablets, including coated tablets, dragees, elixirs, suspensions, liquids, gels, slurries, syrups or inhalations and controlled release forms thereof. Each possibility represents a separate embodiment of the present invention.

Suitable carriers for oral administration are well known in the art. Compositions for oral use may be made using a solid excipient, optionally grinding the resulting mixture, and processing the mixture of granules, after adding suitable auxiliaries as desired, to obtain tablets or dragee cores. Non-limiting examples of suitable excipients include fillers such as sugars, including lactose, sucrose, mannitol, or sorbitol, cellulose preparations such as, maize starch, wheat starch, rice starch, potato starch, gelatin, gum tragacanth, methyl cellulose, hydroxypropylmethylcellulose, and sodium carbomethylcellulose, and/or physiologically acceptable polymers such as polyvinylpyrrolidone (PVP). Each possibility represents a separate embodiment of the present invention.

If desired, disintegrating agents, such as cross-linked polyvinyl pyrrolidone, agar, or alginic acid or a salt thereof, such as sodium alginate, may be added to the composition. Capsules and cartridges of, for example, gelatin for use in a dispenser may be formulated containing a powder mix of the at least one FABAC and a suitable powder base, such as lactose or starch.

Solid dosage forms for oral administration may include capsules, tablets, pill, powders, and granules. In such solid dosage forms, the active ingredient may be admixed with at least one inert pharmaceutically acceptable carrier such as sucrose, lactose, or starch. Such dosage forms may also comprise additional substances other than inert diluents, e.g., lubricating, agents such as magnesium stearate. In the case of capsules, tablets and pills, the dosage forms may also comprise buffering, agents. Tablets and pills may additionally be prepared with enteric coatings. The term "enteral coating", as used herein, refers to a coating which controls the location of composition absorption within the digestive system.

Liquid dosage forms for oral administration may further contain adjuvants, such as wetting agents, emulsifying and suspending agents, and sweetening, flavoring and perfuming agents.

### Additives

According to some embodiments, the composition of the present invention further comprises at least one additive selected from the group consisting of: a diluent, a preservative, an abrasive, an anticaking agent, an antistatic agent, a binder, a buffer, a dispersant, an emollient, an emulsifier, a co-emulsifiers, a humectant or emollient agent, a fiberous material, a film forming agent, a fixative, a foaming agent, a foam stabilizer, a foam booster, a gellant, a lubricant, a moisture barrier agent, a plasticizer, a preservative, a propellant, a stabilizer, a surfactant, a suspending agent, a thickener, a chelating agent, a sequestering agent, a conditioning agent, a wetting agent, a liquefier and a combination thereof. Each possibility represents a separate embodiment of the present invention. According to some embodiments, the at least one additive is suitable for topical administration. According to some embodiments, the at least one additive is suitable for oral administration.

For any agent, combination of agents and composition used within the scope of the invention, the dermatologically effective amount or dose may be estimated initially from cell culture assays. In a non-limiting example, a dose can be formulated in animal models to achieve a circulating concentration range that includes the IC₅₀ as determined in cell culture (e.g., the concentration of the test compound, which achieves a half-maximal inhibition of the epidermal cells proliferation). Such information can be used to more accurately determine useful doses in humans.

Other examples of additives for topical compositions may include sunscreen agents and tanning agents. Sunscreen agents may include those materials commonly employed to block ultraviolet light. Illustrative compounds are the derivatives of PABA, cinnamate and salicylate. For example, octyl methoxycinnamate and 2-hydroxy-4-methoxy benzophenone (also known as oxybenzone) can be used. Octyl methoxycinnamate and 2-hydroxy-4-methoxy benzophenone are also known as parsol MCX and benzophenone-3, respectively. The amount of sunscreen agents employed in the compositions can vary depending upon the degree of UV radiation protection desired. The sunscreen agent added to the composition must be compatible with the active compound (i.e., the at least one FABAC) but in general the composition may comprise from about 1% to about 20% of sunscreen agent. Exact amounts may vary depending upon the sunscreen agent chosen and the desired Sun Protection Factor (SPF).

The composition of the present invention may further comprise an antioxidant/radical scavenger. The inclusion of an anti-oxidant/radical scavenger may increase the benefits of the composition. The anti-oxidant/radical scavenger may be added to the compositions of the present invention in a concentration range of about 0.1% to about 10% total weight of the composition. Anti-oxidants/radical scavengers include, but are not limited to, ascorbic acid (vitamin C) and its salts, and tocopherol (vitamin E).

The composition of the present invention may further comprise at least one additional anti-acne agent other than the FABACs of the invention. Non-limiting examples of known anti-acne agents include: azaleic acid, salicylic acid, benzoylperoxide and sulphur compounds. Certain vitamin A metabolites, as well as agonists, derivatives and prodrugs of vitamin A, may be incorporated into the compositions of the present invention. Examples of vitamin A agents that may be useful in the context of the present invention include, without limitation, the well-known variety of retinol, retinoic acid and retinoic acid receptor (RAR) agonists. Each possibility represents a separate embodiment of the present invention. RAR agonists may include, without limitation, chromans, thiochromans, tetrahydroquinolines, substituted tetrahydronaphthalenes, substituted dihydronaphthalenes, trisubstituted phenyls, aromatic tetracyclic compounds, substituted cyclohexanes, substituted cyclohexenes, substituted cyclohexanedienoic acids, substituted adamentanes, substituted diaryl, heteroaryl compounds, combinations thereof and many more. Each possibility represents a separate embodiment of the present invention.

Vitamin C, or ascorbic acid is a very potent antioxidant and may even be protective against UVA and UVB rays. Studies suggest that topical vitamin E, particularly alpha tocopherol (a form of vitamin E) cream decreased skin roughness, length of facial lines, and wrinkle depth. Studies on mice have also reported reductions in UV-induced skin cancer with its use. Vitamin K may also be useful for treating capillary damage. According to some embodiments, the composition further includes at least one of: vitamin C, vitamin E, vitamin K and any combination thereof. Each possibility represents a separate embodiment of the present invention.

Green and black tea and extracts thereof may be suitable as additives to the composition of the invention. Other plant derived agents which may be used as additives to the composition include, but are not limited to, pomegranate and soy extracts, aloe, ginger, grape seed extract, and coral extracts.

Certain copper containing compounds may both protect skin and help repair it. Of note, copper itself is a toxic metal and it should only be used in products that contain that bind to copper.

Color correctors may be suitable additives to the topical compositions of the invention and may be desired when blemishes are prominent. For example, green neutralizers may mask red lesions; yellow may camouflage dark circles and bruises; and white may help to minimize apparent wrinkles. Other possible additives include glycosaminoglycans, such as hyaluronic acid and the like.

### Product Packaging and Kits

In use, a small quantity of the topical composition, for example from about 0.1 ml to about 100 ml, may be applied to exposed areas of the skin from a suitable container or applicator and, if necessary, it may then be spread over and/or rubbed into the skin using the hand, fingers or a suitable device. According to some embodiments, the composition may be specifically formulated for use as a hand or facial treatment.

When formulated the composition may be packaged in a suitable container to suit its viscosity and intended use by the consumer. For example, a topical composition formulated as a lotion or cream can be packaged in a bottle, or a propellant-driven aerosol device or a container fitted with a pump suitable for finger operation. When the composition is formulated as a cream, it may simply be stored in a non-deformable bottle or squeeze container, such as a tube or a lidded jar. The invention accordingly also provides, according to some embodiments, a closed container containing the composition as herein defined. The shape of the container is not limited in this invention, and can be a tube, a pump dispenser, a compressed dispenser, a bottle, a spray, a sachet or the like.

In some embodiments, the compounds or compositions of the invention are provided in packs in a form ready for administration. In other embodiments, the compounds or compositions are provided in concentrated form in packs, optionally with the diluent required to make final solution(s) for administration. In still other embodiments, the product contains a compound useful in the invention in solid form and, optionally, a separate container with a suitable solvent or carrier for the compound useful in the invention.

According to some embodiments, the present invention provides a kit comprising the composition of the invention in a first suitable container. According to some embodiments, the present invention provides a kit comprising at least one FABAC of the invention in a first suitable container. According to some embodiments, the kit further comprises at least one container other than the first container. According to some embodiments, said at least one other container comprises at least one diluent, excipient, carrier, solvent or additive. Each possibility represents a separate embodiment of the present invention. According to some embodiments, the composition of the invention is formed by combining the content of a first container comprising at least one FABAC and the content of said at least one other container. According to some embodiments, the kit further comprises instructions for use and/or preparation of the composition of the invention. Each possibility represents a separate embodiment of the present invention.

In still other embodiments, the above packs/kits include other components, e.g., instructions for dilution, mixing and/or administration of the product, other containers, syringes, needles, etc. Each possibility represents a separate embodiment of the present invention. Other such pack/kit components will be readily apparent to one of skill in the art.

The following examples are presented in order to more fully illustrate some embodiments of the invention. They should, in no way be construed, however, as limiting the broad scope of the invention.

### EXAMPLES

### Example 1. Cytotoxicity assay

### Study Overview

Full-thickness Epiderm cultures (EFT-400, MatTek) were used to determine toxicity in response to topical application of a test material (Steamchol). A previous viability assay was conducted at concentrations of 40.0, 13.3, 4.4, 1.5 ug/mL and vehicle alone (DMSO); no toxicity was observed (data not shown).

### Experimental Procedures

The test material was provided as a powder. Test solutions were prepared by adding the appropriate amount of powdered material to 1 mL DMSO. Lower concentrations were made by serial dilution with 100% DMSO. The final test material concentrations and treatments were: 2% Steamchol (20,000 ug/mL); 1% Steamchol (10,000 ug/mL); 0.1% Steamchol (1000 ug/mL); 0.01% Steamchol (100 ug/mL); vehicle control (100% DMSO); and untreated control.

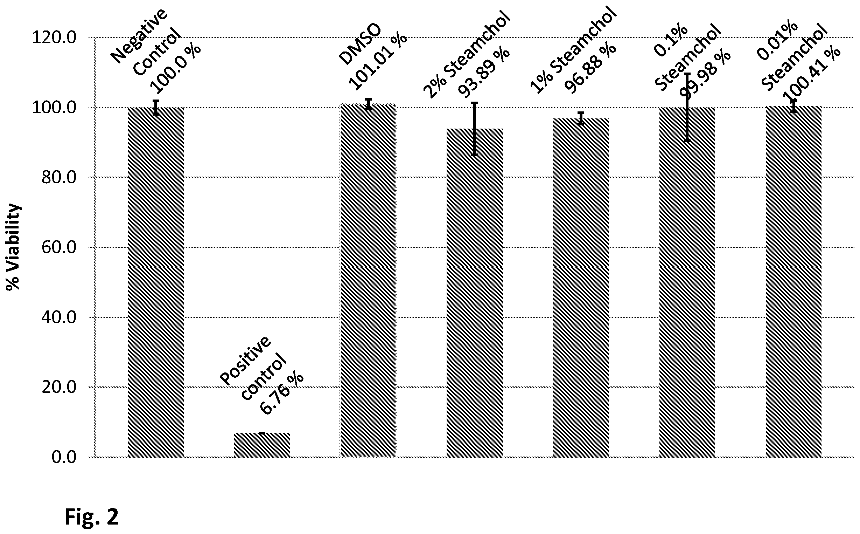

Full-thickness skin cultures (EFT-400) were maintained according to MatTek protocol; the cultures were equilibrated at 37°C and 5% CO₂ for 24 hours prior to application of test materials. Test material was applied to the surface of each culture using sterile techniques (10 µL applied to each culture). Two cultures were assigned to each treatment group and the viability percentages presented below are avarages of viability of each two cultures. Two EFT cultures served as an untreated control (also referred to as negative control in Figure 2), and two other EFT cultures served as a positive control (100 µL 1% Triton X-100).

Following a 24-hour incubation period with the test material, cultures were processed for cell toxicity analysis using an MTT [(3-4,5-dimethylthiazole-2-yl) 2,5-diphenyltetrazoliumbromide] assay (reagents purchased from MatTek). Viable tissues converted MTT into a blue formazan salt that was detected by measuring absorbance at a specific wavelength of light (A₅₇₀). Percent viability was calculated by comparing the A₅₇₀ reading of the test cultures to the A₅₇₀ of the untreated control culture, using the following formula: [Treated A₅₇₀ / Untreated A₅₇₀] * 100

### Results

None of the concentrations tested produced any significant effects on cell viability (Figure 2; Table 2). Cell viability was near 100% compared to the untreated control for all dilutions of Steamchol tested. These results show an advantageous affect of FABACs over retinoids which have been shown to be cytotoxic for fibroblasts and epithelial cells in the range of 0.6-3*10⁵ M (Varani et al. Journal of Investigative Dermatology (1993) 101, 839-842), and increase epithelial cell death (Ding et al. Invest Ophthalmol Vis Sci. 2013 Jun 26;54(6):4341-50).

**Table 2. Cell Viability following a 24-hour exposure to steamchol**

| **Test compound** | **Cell Viability** |
|---|---|
| Untreated Cells (Negative Control) | 100% |
| Positive control | 6.8 % |
| DMSO vehicle | 101.0 % |
| 2% Steamchol | 93.89 % |
| 1% Steamchol | 96.88% |
| 0.1% Steamchol | 99.98 % |
| 0.01% Steamchol | 100.41 % |

### Example 2. Effect of Steamchol or Aramchol on cholesterol efflux and ABCA1 mRNA and protein levels

Steamchol was incubated with human skin fibroblasts for 20 hours to measure cholesterol loading in the presence of [³H] cholesterol. After series of washing and adding efflux containing cholesterol acceptors, medium was collected and centrifuged and cell associated cholesterol was compared to effluxed cholesterol in the presence and absence of the similar FABAC. This research project also included quantification of mRNA of ABCA1 and direct measurement of ABCA1. Results demonstrated that cholesterol efflux in fibroblasts was significantly enhanced and that ABCA1 protein concentration increased approximately 2 fold when efflux took place in the presence of FABAC as compared to untreated cells.

Similarly, cells were pre-incubated with [³H] cholesterol, washed four times and then placed in an incubation medium with or without Aramchol for 20 hours. Following the incubation, radioactivity was measured separately in the medium and in the cells. Cholesterol efflux percentage was calculated as radioactivity in medium divided by the total radioactivity (cells+medium). Results demonstrated that cholesterol efflux in fibroblasts was significantly enhanced and that ABCA1 protein concentration increased approximately 2 fold when efflux took place in the presence of Aramchol as compared to untreated cells.

### Example 3. Effect of Steamchol on gene expression in skin culture

A full-thickness in vitro skin culture model, Epiderm FT (MatTek, MA), was treated with Steamchol. A non-cytotoxic concentration of Steamchol in DMSO (0.5%, 5000 µg/mL) was applied to the surface of each test culture and cells were collected 24 hours post-application. Control cells were similarly treated with DMSO without Steamchol. Tissues were collected in RNAlater for gene expression analysis. Gene expression was analyzed using validated Taqman gene expression assays in a Taqman Low Density Array (TLDA) format. 94 genes that regulate a variety of known functions in skin were analyzed, including the ABCA1 and SCD1 genes. The experimental set up was conducted in a 96-well format using validated Taqman gene expression assays. Each gene was assayed in duplicates. Statistics were carried out using the StatMiner software v4.2 (unpaired t-tests, p<0.05, N=4) to compare the 0.5% Steamchol group to the DMSO control group.

The effect of Steamchol on gene expression in the Epiderm FT culture revealed that out of the 94 selected genes in the panel, two genes, KRT 1 (keratin 1) and KRT 10 (keratin 10) demonstrated statistically significant deviation of more than two fold from cells treated with DMSO (see Table 3 below). No significant change in mRNA levels of ABCA1 was observed.

**Table 3: Fold change of keratin 1 and 10 gene expression after treatment with FABAC**

| Gene ID | Gene Name | Fold Change |
|---|---|---|
| KRT10 | keratin 10 | -2.60 |
| KRT1 | keratin 1 | -2.49 |

As shown in table 3, Steamchol significantly inhibits the expression of keratins 1 and 10 which are known markers for keratinocyte differentiation. The decrease in rate of differentiation can attenuate cornification rate of keratinocytes to corneocytes and relieve pore clogging of the pilosebaceous opening, thus allowing more effective sebum drainage. This effect is similar to that of retinoic acid and salicylic acid used for treatment of acne.

### Example 4. Effect of FABACs on keratinocyte differentiation

In order to examine the effect of the disclosed FABACs on differentiation of keratinocytes, the highly differentiated full-thickness in vitro skin culture model Epiderm FT (MatTek, MA) is used. Duplicates of skin cultures are treated with three different concentrations of Steamchol in DMSO and three different concentrations of Aramchol in DMSO. One of the tested concentrations is 0.5% (5000 µg/mL) which was efficient in reducing gene expression of KRT1 and KRT10. Two non-treated skin cultures and two cultures treated with vehicle alone (DMSO) are used as negative controls. Two skin cultures treated with retinoic acid are used as a positive control.

The cells are collected 24 hours following application of the compositions to the surface of each test culture. Proteins are extracted from part the cells and subjected to Western Blot and ELISA analyses using primary antibodies specific for Keratin 1, Keratin 10, SCD1 and ABCA-1. Another part of the cells is fixated and subjected to immunohistochemical staining using primary antibodies specific for Keratin 1, Keratin 10, SCD1 and ABCA-1.

Down-regulation in expression of Keratin 1 and/or Keratin 10 is indicative of a decrease in keratinocyte differentiation.

### Example 5. Effect of FABACs on P. acne behavior

Propionibacterium acnes (P. acnes) is a commensal organism that thrives in the presence of excess sebum and plays a key role in acne vulgaris. An obstacle associated with the resistance of p.acne to treatment is its propensity to develop microfilm colonies. P. acnes strains isolated from acne patients form biofilms in vitro that are characterized by increased lipase activity as compared to planktonic organisms. Lipase may be, at least in part, responsible for the production of irritant fatty acids that promote inflammation and therefore for the itch sensation at the comedone.

In order to examine the effect of FABACs on P. acnes behavior, a few commercial and/or clinically derived P. ances strains are used. FABACs (Steamchol and/or Aramchol) are used to treat bacterial cultures and the following parameters are examined: Minimum Inhibitory Concentration (MIC) of bacterial growth, Time to Kill, the Minimum Biofilm Eradication Concentration (MBECTM), lipase activity and morphological changes of bacteria. As positive controls, the following substances are administered to corresponding bacterial cultures: erythromycin, clindamycin, tetracycline, salicylic acid and chlorhexidine.

### Example 6. Examination of the anti-inflammatory effect of FABACs

A highly differentiated full-thickness in vitro skin culture model (Epiderm FT, MatTek, MA) is used to examine the anti-inflammatory effect of FABACs. The skin cultures are treated with oxidized squalene or phorbol esters to induce inflammation and the inflammation is verified by examining IL-8 levels.

Next, duplicates of skin cultures are treated with several different concentrations of FABACs (Steamchol or Aramchol in DMSO). Two non-treated skin cultures and two cultures treated with vehicle alone (DMSO) are used as negative controls. In order to examine the anti-inflammatory effect of FABACs, the expression levels of IL-1, IL-6, IL-8, IL-16 and NFkB is examined at the mRNA and protein level. In addition, enzymatic inhibition is examined for COX-1, COX-2, PGE₂ and PLA₂. Skin cultures treated with ibuprofen or indomethacin are used as positive controls for enzyme inhibition.

## Claims

1. A composition for use in a method of treating a skin condition associated with altered sebum levels, the composition comprising as an active ingredient a fatty acid bile acid conjugate (FABAC) and a pharmaceutically acceptable diluent, carrier or excipient, wherein the FABAC has the formula I:
W - X - G (I)
wherein G represents a bile acid or a bile salt radical; W represents one or two fatty acid radicals having 6-22 carbon atoms; and X represents a bonding member comprising a heteroatom or a direct C-C or C=C bond.

2. The composition for use according to claim 1, wherein the FABAC comprises two fatty acid radicals wherein at each occurrence W is independently a fatty acid radical having 6-22 carbon atoms; and X is independently a bonding member comprising a heteroatom or a direct C-C or C=C bond.

3. The composition for use according to claim 1-2, wherein said bonding member is selected from the group consisting of: NH, P, S, O, or a direct C-C or C=C bond, preferably wherein said bonding member is NH.

4. The composition for use according to any one of claims 1-3, wherein said one or two fatty acids are independently selected from the group consisting of: stearic acid, behenic acid, arachidylic acid, palmitic acid, arachidonic acid, eicosapentaenoic acid and oleic acid.

5. The composition for use according to claim 4, wherein said one or two fatty acid radicals is a radical of arachidylic acid.

6. The composition for use according to any one of claims 1-4, wherein said bile acid is selected from the group consisting of: cholic acid, ursodeoxycholic acid, chenodeoxycholic acid, deoxycholic acid, lithocholic acid, and derivatives thereof.

7. The composition for use according to claim 6, wherein said bile acid is a cholic acid.

8. The composition for use according to claim 1, wherein said FABAC is selected from the group consisting of:
3-beta-stearoyl-amido,7α,12α-dihydroxy-5-beta-cholan-24-oic acid, 3-beta-arachidylamido, 7α,12α-dihydroxy-5-beta-cholan-24-oic acid, and a combination thereof.

9. The composition for use according to any one of claims 1-8, wherein the skin condition associated with altered sebum levels is selected from the group consisting of: acne, seborrhea, seborrhoeic dermatitis, a sebaceous cyst, sebaceous hyperplasia, dandruff and folliculitis.

10. The composition for use according to claim 9, wherein the skin condition associated with altered sebum levels is acne, preferably acne is acne vulgaris.

11. The composition for use according to any one of claims 1-9, wherein the condition associated with altered sebum levels is a skin condition associated with elevated sebum levels.

12. The composition for use according to any one of claims 1-11 formulated for oral administration.

13. The composition for use according to any one of claims 1-11 formulated for topical administration.

14. The composition for use according to claim 13, wherein said composition formulated for topical administration is formulated in a form selected from the group consisting of: aqueous solution, cream, lotion, water in oil or oil in water emulsion, multiple emulsion, silicone emulsion, microemulsion, nanoemulsion, gel, and an aqueous solution with a co-solvent.

15. The composition for use according to any one of claims 1-14, wherein said composition further comprises one or more of an additional anti-acne agent selected from:
a. azaleic acid, salicylic acid, benzoylperoxide, Sulphur compounds;
b. vitamin A metabolites, agonists, derivatives, and prodrugs, preferably retinol, retinoic acid, and retinoic acid receptor (RAR) agonists;
c. vitamin C, vitamin E, vitamin K;
d. green tea, black tea, pomegranate, soy, aloe, ginger, grape, or coral extracts;
e. copper containing compounds;
f. color correctors; or
g. glycoaminoglycans, preferably hyaluronic acid.

## Patentansprüche

1. Zusammensetzung zur Verwendung bei einem Verfahren zum Behandeln eines Hautzustands, der mit veränderten Sebumspiegeln einhergeht, wobei die Zusammensetzung als aktiven Inhaltsstoff ein Fettsäure-Gallensäure-Konjugat (FABAC) umfasst sowie ein pharmazeutisch annehmbares Lösungsmittel und Träger- oder Hilfsstoff, wobei das FABAC die Formel I hat:
W-X-G (I),
wobei G für eine Gallensäure oder einen Gallensäuresalzradikal steht; W für einen oder zwei Fettsäureradikale mit 6-22 Kohlenstoffatomen steht; und X für ein Bindungsglied steht, das ein Heteroatom oder eine direkte C-C- oder C=C-Bindung umfasst.

2. Zusammensetzung zur Verwendung nach Anspruch 1, wobei das FABAC zwei Fettsäureradikale umfasst, und wobei W bei jedem Vorkommen jeweils unabhängig ein Fettsäureradikal mit 6-22 Kohlenstoffatomen ist; und X jeweils unabhängig ein Bindungsglied ist, das ein Heteroatom oder eine direkte C-C- oder C=C-Bindung umfasst.

3. Zusammensetzung zur Verwendung nach Anspruch 1 bis 2, wobei das Bindungsglied aus der Gruppe bestehend aus den folgenden ausgewählt ist: NH, P, S, O oder eine direkte C-C- oder C=C-Bindung, wobei das Bindungsglied bevorzugt NH ist.

4. Zusammensetzung zur Verwendung nach einem der Ansprüche 1 bis 3, wobei die eine oder beide Fettsäuren jeweils unabhängig aus der Gruppe bestehend aus den folgenden ausgewählt ist/sind: Stearinsäure, Behensäure, Arachidinsäure, Palmitinsäure, Arachidonsäure, Eicosapentaensäure und Oleinsäure.

5. Zusammensetzung zur Verwendung nach Anspruch 4, wobei ein oder beide Fettsäureradikale ein Radikal von Arachidinsäure ist/sind.

6. Zusammensetzung zur Verwendung nach einem der Ansprüche 1 bis 4, wobei die Gallensäure aus der Gruppe bestehend aus Cholsäure, Ursodeoxycholsäure, Chenodeoxycholsäure, Deoxycholsäure, Lithocholsäure und Derivaten davon ausgewählt ist.

7. Zusammensetzung zur Verwendung nach Anspruch 6, wobei die Gallensäure eine Cholsäure ist.

8. Zusammensetzung zur Verwendung nach Anspruch 1, wobei das FABAC aus der Gruppe bestehend aus:
3-Betastearoylamido, 7α,12α-Dihydroxy-5-betacholan-24-säure, 3-Betaarachidylamino, 7α,12α-Dihydroxy-5-betacholan-24-säure und einer Kombination davon ausgewählt ist.

9. Zusammensetzung zur Verwendung nach einem der Ansprüche 1 bis 8, wobei der Hautzustand, der mit veränderten Sebumspiegeln einhergeht, aus der Gruppe bestehend aus Akne, Seborrhö, seborrhoische Dermatitis, einer Talgzyste, Talgdrüsenhyperplasie, Haarschuppen und Follikulitis ausgewählt ist.

10. Zusammensetzung zur Verwendung nach Anspruch 9, wobei der Hautzustand, der mit veränderten Sebumspiegeln einhergeht, Akne ist, wobei die Akne bevorzugt Akne vulgaris ist.

11. Zusammensetzung zur Verwendung nach einem der Ansprüche 1 bis 9, wobei der Zustand, der mit veränderten Sebumspiegeln einhergeht, ein Hautzustand ist, der mit erhöhten Sebumspiegeln einhergeht.

12. Zusammensetzung zur Verwendung nach einem der Ansprüche 1 bis 11, wobei die Zusammensetzung für eine orale Verabreichung formuliert ist.

13. Zusammensetzung zur Verwendung nach einem der Ansprüche 1 bis 11, wobei die Zusammensetzung für eine topische Verabreichung formuliert ist.

14. Zusammensetzung zur Verwendung nach Anspruch 13, wobei die Zusammensetzung, die für eine topische Verabreichung formuliert ist, in einer Form formuliert wird, die aus der Gruppe bestehend aus den folgenden ausgewählt ist: wässriger Lösung, Creme, Lotion, Wasser in Öl oder Öl in einer Wasseremulsion, Multiple-Emulsion, Silikonemulsion, Mikroemulsion, Nanoemulsion, Gel und auswässriger Lösung mit einem Co-Lösungsmittel.

15. Zusammensetzung zur Verwendung nach einem der Ansprüche 1 bis 14, wobei die Zusammensetzung ferner einen oder mehrere zusätzliche Anti-Aknemittel umfasst, die aus den folgenden ausgewählt sind:
a. Azelainsäure, Salicylsäure, Benzoylperoxid, Schwefelverbindungen;
b. Vitamin-A-Metaboliten, Agonisten, Derivate und Prodrugs, bevorzugt Retinol, Retinsäure und Retinsäure-Rezeptor-Agonisten (RAR-Agonisten);
c. Vitamin C, Vitamin E, Vitamin K;
d. Extrakte aus grünem Tee, schwarzem Tee, Granatapfel, Soja, Aloe, Ingwer, Traube oder Koralle;
e. Kupferhaltige Verbindungen;
f. Farbkorrigierer; oder
g. Glycoaminglycane, bevorzugt Hyaluronsäure.

## Revendications

1. Composition destinée à être utilisée dans un procédé de traitement d'une affection de la peau associée à une altération des taux de sébum, la composition comprenant comme ingrédient actif un conjugué d'acide biliaire d'acide gras (CABAG) et un diluant, support ou excipient acceptable d'un point de vue pharmaceutique, dans laquelle CABAG a pour formule I :
W - X - G (I)
dans laquelle G représente un acide biliaire ou un radical de sel biliaire ; W représente un ou deux radicaux acides gras comportant 6 à 22 atomes de carbone : et X représente un élément de liaison comprenant un hétéroatome ou une liaison directe CC ou C=C

2. Composition à utiliser selon la revendication 1, dans laquelle le CABAG comprend deux radicaux acide gras, dans lesquels à chaque occurrence, W est indépendamment un radical acide gras ayant 6 à 22 atomes de carbone ; et X est indépendamment un élément de liaison comprenant un hétéroatome ou une liaison directe CC ou C=C.

3. Composition à utiliser selon les revendications 1 et 2, dans laquelle ledit élément de liaison est choisi dans le groupe constitué de : NH, P, S, O, ou une liaison directe CC ou C=C, de préférence dans laquelle ledit élément de liaison est NH.

4. Composition à utiliser selon l'une quelconque des revendications 1 à 3, dans laquelle ledit ou lesdits deux acides gras sont indépendamment choisis dans le groupe constitué de : l'acide stéarique, l'acide béhénique, l'acide arachidylique, l'acide palmitique, l'acide arachidonique, l'acide eicosapentaénoïque et l'acide oléique.

5. Composition à utiliser selon la revendication 4, dans laquelle ledit un ou deux radicaux acides gras est un radical d'acide arachidylique.

6. Composition à utiliser selon l'une quelconque des revendications 1 à 4, dans laquelle ledit acide biliaire est choisi dans le groupe comprenant : l'acide cholique, l'acide ursodésoxycholique, l'acide chénodésoxycholique, l'acide déoxycholique, l'acide lithocholique et leurs dérivés.

7. Composition à utiliser selon la revendication 6, dans laquelle ledit un acide cholique.

8. Composition à utiliser selon la revendication 1, dans laquelle ledit CABAG est choisi dans le groupe constitué de :
l'acide 3-bêta-stéaroyl-amido, 7α, 12α-dihydroxy-5-bêta-cholan-24-oique, l'acide 3-bêta-arachidylamido, 7α, 12α-dihydroxy-5-bêta-cholan-24-oique, et leur combinaison.

9. Composition à utiliser selon l'une quelconque des revendications 1 à 8, dans laquelle l'état cutané associé à des taux de sébum modifiés est choisi dans le groupe comprenant : acné, séborrhée, dermatite séborrhéique, kyste sébacé, hyperplasie sébacée, pellicules et folliculite.

10. Composition à utiliser selon la revendication 9, dans laquelle l'état de la peau associé aux niveaux de sébum modifiés est l'acné, de préférence l'acné est l'acné commune.

11. Composition à utiliser selon l'une quelconque des revendications 1 à 9, dans laquelle l'état associé à des niveaux de sébum modifiés est un état de la peau associé à des niveaux élevés de sébum.

12. Composition à utiliser selon l'une quelconque des revendications 1 à 11, formulée pour une administration par voie orale.

13. Composition à utiliser selon l'une quelconque des revendications 1 à 11, formulée pour une administration par voie topique.

14. Composition à utiliser selon la revendication 13, dans laquelle ladite composition formulée pour administration par voie topique est formulée sous une forme choisie dans le groupe comprenant : une solution aqueuse, une crème, une lotion, une émulsion d'eau dans l'huile ou d'huile dans l'eau, une émulsion multiple, une émulsion de silicone, microémulsion, une nanoémulsion, un gel et une solution aqueuse avec un co-solvant.

15. Composition à utiliser selon l'une quelconque des revendications 1 à 14, dans laquelle ladite composition comprend en outre un ou plusieurs agents anti-acné supplémentaires choisis parmi :
(a) l'acide azaléique, l'acide salicylique, le peroxyde de benzoyle, des composés du soufre ;
(b) des métabolites, des agonistes, des dérivés et des promédicaments de la vitamine A, de préférence du rétinol, de l'acide rétinoïque et des agonistes des récepteurs de l'acide rétinoïque (RAR) ;
(c) la vitamine C, la vitamine E, la vitamine K ;
(d) des extraits de thé vert, de thé noir, de grenade, de soja, d'aloès, de gingembre, de raisin ou de corail ;
(e) des composés contenant du cuivre ;
(f) des correcteurs de couleur ; ou
(g) des glycoaminoglycanes, de préférence de l'acide hyaluronique.
